# EUROPEAN PATENT APPLICATION

(11) **EP 4 492 388 A1**
(43) Date of publication of application: **15.01.2025**
(21) Application number: 23777735.4
(22) Date of filing: 28.02.2023
(51) Int. Cl.: G16C 10/00

(54) **MOLECULE GENERATION METHOD AND RELATED DEVICE**

(30) Priority: 31.03.2022 CN 202210334013
(71) Applicant: Huawei Cloud Computing Technologies Co., Ltd., Guiyang, Guizhou 550025 (CN)
(72) Inventor: QIAO, Nan, Guiyang, Guizhou 550025 (CN); LIN, Xinyuan, Guiyang, Guizhou 550025 (CN); PAN, Ruiqing, Guiyang, Guizhou 550025 (CN); XIONG, Zhaoping, Guiyang, Guizhou 550025 (CN); WANG, Chunjie, Guiyang, Guizhou 550025 (CN)
(74) Representative: Gill Jennings & Every LLP
(86) International application number: PCT/CN2023/078884
(87) International publication number: WO 2023/185357

(57) **Abstract**

A molecule generation method and a related apparatus are provided. The molecule generation method includes: receiving a constraint condition entered by a user on a terminal, where the constraint condition indicates a condition that a property of a molecule needs to meet, and the property of the molecule includes any one or more of a molecular weight, water solubility, lipid solubility, bioactivity, synthesizability, a docking binding affinity for a specific molecule, a similarity to a source molecule, an included specific substructure, pharmacokinetics, and toxicity of the molecule; generating a first molecule set based on the constraint condition, where the first molecule set includes one or more molecules; and returning information about the one or more molecules in the first molecule set to the terminal. The molecule generation method is used to generate molecules, to improve molecule generation efficiency and reduce costs such as time costs and manpower, material, and financial resources.

## Description

This application claims priority to Chinese Patent Application No. 2022103340137, filed with the China National Intellectual Property Administration on March 31, 2022 and entitled "MOLECULE GENERATION METHOD AND RELATED APPARATUS", which is incorporated herein by reference in its entirety.

### TECHNICAL FIELD

This application relates to the field of drug research and development, and in particular, to a molecule generation method and a related apparatus.

### BACKGROUND

New drug research and development need to go through stages of drug discovery, preclinical research, clinical trial, and the like. A conventional drug discovery process is as follows: Pharmaceutical chemists manually design a molecule (for example, a pilot compound) based on accumulated prior knowledge, optimize a structure of the molecule to obtain a large quantity of new compound molecules that have better drug characteristics or meet specific requirements, and then study physicochemical properties, metabolic properties, toxicological data, and the like of the compound molecules through experiments, to select an optimal compound molecule that meets druglikeness. However, this process takes a long time and costs a lot.

### SUMMARY

This application provides a molecule generation method and a related apparatus. The molecule generation method provided in this application is used to generate molecules, to improve molecule generation efficiency and reduce costs such as time costs and manpower, material, and financial resources.

According to a first aspect, this application provides a molecule generation method, including: receiving a constraint condition entered by a user on a terminal, where the constraint condition indicates a condition that a property of a molecule needs to meet, and the property of the molecule includes any one or more of a molecular weight, water solubility, lipid solubility, bioactivity, synthesizability, a docking binding affinity for a specific molecule, a similarity to a source molecule, an included specific substructure, pharmacokinetics, and toxicity of the molecule; generating a first molecule set based on the constraint condition, where the first molecule set includes one or more molecules; and returning information about the one or more molecules in the first molecule set to the terminal.

It can be learned that according to the molecule generation method provided in this application, the user only needs to enter the constraint condition on the terminal based on a requirement, to obtain one or more molecules required by the user. The constraint condition indicates the condition that the property of the molecule needs to meet. This not only reduces manpower, material, and financial resources, but also improves molecule research and development efficiency. In addition, operations are convenient, and applicability is high.

Based on the first aspect, in a possible implementation, the constraint condition includes a first constraint condition, and the generating a first molecule set based on the constraint condition includes a plurality of iterations, where in each iteration, the method includes: generating a second molecule set based on a current generation parameter, where the second molecule set includes one or more molecules; determining a property of each molecule in the second molecule set; and scoring each molecule in the second molecule set based on the first constraint condition, to obtain a scoring result of each molecule in the second molecule set, where the scoring result of each molecule in the second molecule set is used to adjust a generation parameter in a next iteration, where the first molecule set includes the molecules in the second molecule set generated in each iteration, or the first molecule set is a molecule in a second molecule set generated in a last iteration.

It can be learned that the method provided in this application not only has a function of generating a molecular formula, but also can optimize molecules. The molecules are optimized through a plurality of iterations based on a first constraint condition entered by the user, where the first constraint condition is entered by the user based on a specific requirement. Specifically, in a current iteration, molecules are generated based on a current generation parameter, a property of each molecule is determined, each molecule is scored based on the first constraint condition, where a scoring result of each molecule is used to adjust a generation parameter in a next iteration, and in the next iteration, new molecules are generated based on an adjusted generation parameter, ..., to finally obtain a molecule that meets the first constraint condition and has a relatively high score, that is, a molecule that can better meet the user requirement. In different application scenarios, requirements are different. The user may set and enter a first constraint condition according to a specific application scenario, to obtain a molecule that meets the user requirement. The molecule generation method provided in this application is easy to operate and has high applicability.

Based on the first aspect, in a possible implementation, the constraint condition includes a second constraint condition and a third constraint condition, and the generating a first molecule set based on the constraint condition includes a plurality of iterations, where in each iteration, the method includes: generating a third molecule set based on a current generation parameter, where the third molecule set includes one or more molecules; determining a property of each molecule in the third molecule set; filtering the molecules in the third molecule set based on the second constraint condition, and retaining a molecule when the molecule meets the second constraint condition, or discarding a molecule when the molecule does not meet the second constraint condition, to obtain a fourth molecule set; and scoring each molecule in the fourth molecule set based on the third constraint condition, to obtain a scoring result of each molecule in the fourth molecule set, where the scoring result of each molecule in the fourth molecule set is used to adjust a generation parameter in a next iteration, where the first molecule set includes a molecule in the fourth molecule set obtained in each iteration, or the first molecule set is a molecule in a fourth molecule set obtained in a last iteration.

It can be seen that, before scoring, generated molecules are filtered based on an entered constraint condition, to filter out some molecules that do not meet a requirement, molecules retained through filtering are scored, a generation parameter in a next iteration is adjusted based on scoring results, new molecules are generated based on an adjusted generation parameter, ..., and in the same manner, a plurality of iterations are implemented, to finally obtain a molecule that meets the user requirement. Implementing this application can improve molecule generation efficiency.

Based on the first aspect, in a possible implementation, the determining a property of each molecule in the second molecule set includes: determining the property of each molecule in the second molecule set based on a molecular structure or a molecular formula of each molecule in the second molecule set; and/or predicting the property of each molecule in the second molecule set based on a trained molecular property prediction model; or the determining a property of each molecule in the third molecule set includes: determining the property of each molecule in the third molecule set based on a molecular structure or a molecular formula of each molecule in the third molecule set; and/or predicting the property of each molecule in the third molecule set based on a trained molecular property prediction model.

It may be understood that some specific substructures have some fixed properties, and the specific substructure may be an atom, a chemical bond, or the like. Therefore, whether a molecule has some fixed properties may be determined according to whether some specific substructures are included in a molecular structure or a molecular formula. In addition, a property of a molecule may be further predicted based on the trained molecular property prediction model.

According to a second aspect, this application provides a molecule generation apparatus, including: a communication module, configured to receive a constraint condition entered by a user on a terminal, where the constraint condition indicates a condition that a property of a molecule needs to meet, and the property of the molecule includes any one or more of a molecular weight, water solubility, lipid solubility, bioactivity, synthesizability, a docking binding affinity for a specific molecule, a similarity to a source molecule, an included specific substructure, pharmacokinetics, and toxicity of the molecule; and a processing module, configured to generate a first molecule set based on the constraint condition, where the first molecule set includes one or more molecules. The communication module is further configured to return information about the one or more molecules in the first molecule set to the terminal.

Based on the second aspect, in a possible implementation, the processing module includes a molecule generation submodule, a molecular property determining submodule, and a molecule scoring submodule. The constraint condition includes a first constraint condition, and the generating a first molecule set based on the constraint condition includes a plurality of iterations, where in each iteration,
the molecule generation submodule is configured to generate a second molecule set based on a current generation parameter, where the second molecule set includes one or more molecules;
the molecular property determining submodule is configured to determine a property of each molecule in the second molecule set; and
the molecule scoring submodule is configured to score each molecule in the second molecule set based on the first constraint condition, to obtain a scoring result of each molecule in the second molecule set, where the scoring result of each molecule in the second molecule set is used to adjust a generation parameter in a next iteration, where
the first molecule set includes the molecules in the second molecule set generated in each iteration, or the first molecule set is a molecule in a second molecule set generated in a last iteration.

Based on the second aspect, in a possible implementation, the processing module includes a molecule generation submodule, a molecular property determining submodule, a molecule filtering submodule, and a molecule scoring submodule. The constraint condition includes a second constraint condition and a third constraint condition, and the generating a first molecule set based on the constraint condition includes a plurality of iterations, where in each iteration,
the molecule generation submodule is configured to generate a third molecule set based on a current generation parameter, where the third molecule set includes one or more molecules;
the molecular property determining submodule is configured to determine a property of each molecule in the third molecule set;
the molecule filtering submodule is configured to filter the molecules in the third molecule set based on the second constraint condition, and retain a molecule when the molecule meets the second constraint condition, or discard a molecule when the molecule does not meet the second constraint condition, to obtain a fourth molecule set; and
the molecule scoring submodule is configured to score each molecule in the fourth molecule set based on the third constraint condition, to obtain a scoring result of each molecule in the fourth molecule set, where the scoring result of each molecule in the fourth molecule set is used to adjust a generation parameter in a next iteration, where
the first molecule set includes a molecule in the fourth molecule set obtained in each iteration, or the first molecule set is a molecule in a fourth molecule set obtained in a last iteration.

Based on the second aspect, in a possible implementation, the molecular property determining submodule is configured to:
determine the property of each molecule in the second molecule set based on a molecular structure or a molecular formula of each molecule in the second molecule set; and/or predict the property of each molecule in the second molecule set based on a trained molecular property prediction model; or
determine the property of each molecule in the third molecule set based on a molecular structure or a molecular formula of each molecule in the third molecule set; and/or predict the property of each molecule in the third molecule set based on a trained molecular property prediction model.

Functional submodules in the second aspect are configured to implement the method described in any one of the first aspect or the possible implementations of the first aspect.

According to a third aspect, this application provides a molecule generation device, including a memory and a processor. The memory is configured to store instructions, and the processor is configured to invoke the instructions stored in the memory to perform the method described in the first aspect or any one of the possible implementations of the first aspect.

According to a fourth aspect, this application provides a computer storage medium, including program instructions. When the program instructions are run on a computer, the computer is enabled to perform the method described in the first aspect or any one of the possible implementations of the first aspect.

According to a fifth aspect, this application provides a computer program product, including program instructions. When the computer program product is executed by a molecule generation device, the molecule generation device performs the method in the first aspect. The computer program product may be a software installation package. When the method provided in any possible implementation of the first aspect needs to be used, the computer program product may be downloaded, and may be executed on the molecule generation device, to implement the method in the first aspect.

### BRIEF DESCRIPTION OF DRAWINGS

FIG. 1 is a diagram of an architecture of a system according to this application;
FIG. 2 is a diagram of a structure of a server according to this application;
FIG. 3 is a diagram of components in a server according to this application;
FIG. 4 is a diagram of a molecule generation time sequence on a server side according to this application;
FIG. 5 is a schematic flowchart of a molecule generation method according to this application;
FIG. 6 is a diagram of a partial procedure of a molecule generation method according to this application;
FIG. 7 is a diagram of a scenario according to this application;
FIG. 8 is a diagram of a molecule generation time sequence on a server side according to this application;
FIG. 9 is a schematic flowchart of another molecule generation method according to this application;
FIG. 10 is a diagram of a partial procedure of another molecule generation method according to this application;
FIG. 11 is a diagram of a structure of a molecular property prediction model according to this application;
FIG. 12 is a diagram of a structure of a molecule generation apparatus according to this application; and
FIG. 13 is a diagram of a structure of a molecule generation device according to this application.

### DESCRIPTION OF EMBODIMENTS

For ease of description and understanding of the solution, in this application, "first", "second", and the like are used to distinguish between same objects rather than specific reference. "/" represents an "or" relationship. For example, A/B represents A or B.

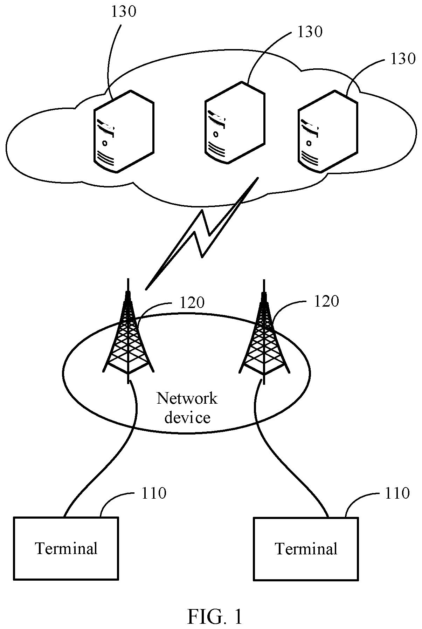

This application provides a system. FIG. 1 is a diagram of an architecture of a system according to this application. The system relates to a terminal 110, a network device 120, and at least one server 130.

The terminal 110 may be an electronic device like a mobile phone, a personal laptop computer, or a desktop computer. The terminal 110 is configured to receive an operation of a user. In this application, a user may enter a constraint condition on the terminal, and correspondingly, the terminal receives the constraint condition entered by the user.

The constraint condition indicates a condition that a property of a molecule needs to meet, and the property of the molecule includes any one or more of a molecular weight, water solubility, lipid solubility, bioactivity, synthesizability, a docking binding affinity for a specific molecule, a similarity to a source molecule, an included specific substructure, pharmacokinetics, and toxicity of the molecule. The pharmacokinetics and toxicity (absorption distribution metabolism excretion and toxicity, ADMET) mean absorption (adsorption), distribution (distribution), metabolism (metabolism), excretion (excretion), and toxicity (toxicity). The source molecule is entered by the user, and the source molecule may be any molecule. A definition of a molecular similarity may be specifically set based on a user requirement. For example, in an implementation, the molecular similarity may be a similarity between two molecular structures. A method for calculating the molecular similarity is not specifically limited in this application. The synthesizability means difficulty of synthesizing a molecule during practical application. A molecular docking binding affinity includes difficulty of docking between molecules, a size of a docking area, a docking position, and the like. The specific molecule may be a target molecule of a disease, or the specific molecule may be another molecule specified by the user. The specific substructure includes one or a combination of a specific chemical bond and a specific atom. Some specific substructures have some specific properties. Therefore, some properties may be determined by determining whether specific substructures are included in a molecule and by determining a quantity of specific substructures.

Therefore, the constraint condition may include any one or more of a condition that the molecular weight needs to meet, a condition that the water solubility needs to meet, a condition that the lipid solubility needs to meet, a condition that the bioactivity needs to meet, a condition that the similarity to the source molecule needs to meet, a condition that the synthesizability needs to meet, a condition that the docking binding affinity for the specific molecule needs to meet, a condition that the pharmacokinetics and toxicity need to meet, whether a specific substructure is included, a quantity of included specific substructures, and the like.

The terminal 110 is further configured to communicate with the server 130 via the network device 120. For example, in this application, the terminal 110 may send, via the network device 120, the constraint condition entered by the user to the server 130.

The network device 120 is configured to communicate data between the terminal 110 and the server 130 over a communication network of any communication mechanism/communication standard. The communication network may be in a form of a wide area network, a local area network, a point-to-point connection, or any combination thereof.

The server 130 may be a computing device located in a cloud, where the cloud may be a private cloud, a public cloud, or a hybrid cloud, and the cloud includes one or more servers 130. In this application, the server 130 is configured to receive data sent by the terminal 110, where the data may be, for example, the constraint condition. The server 130 is further configured to perform processing based on the data sent by the terminal. Specifically, the server 130 may be configured to generate a new molecule based on the constraint condition sent by the terminal 110. The server 130 is further configured to send data to the terminal 110. Specifically, the server 130 may be configured to send the generated new molecule, other information related to the new molecule, and the like to the terminal 110.

Based on the foregoing architecture of the system, this application provides a molecule generation method. For ease of understanding the molecule generation method provided in this application, before method embodiments are described, structures of the server provided in this application and a function of each structure of the server are first described.

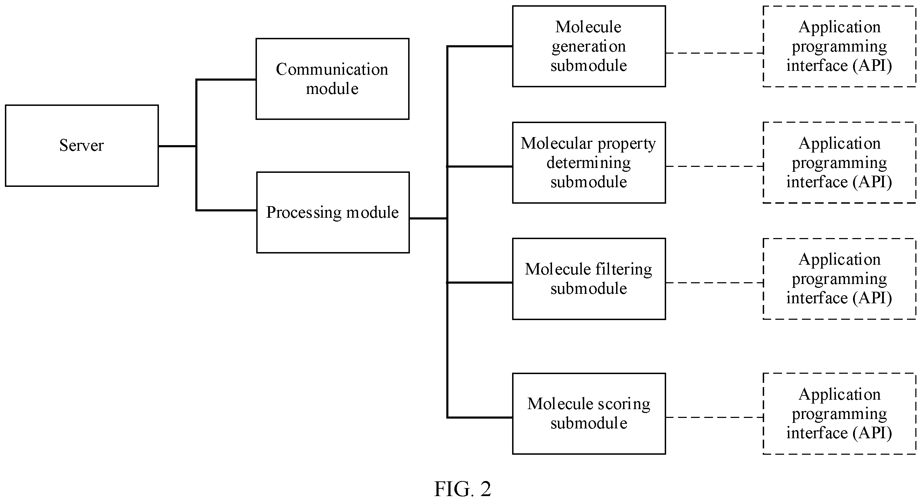

FIG. 2 is a diagram of a structure of a server according to this application. The server includes a communication module and a processing module. The processing module includes a molecule generation submodule, a molecular property determining submodule, a molecule filtering submodule, and a molecule scoring submodule.

In this application, the communication module is configured to receive data or information sent by the terminal. The data or information includes the constraint condition sent by the terminal. The constraint condition in this application includes a first constraint condition, a second constraint condition, and a third constraint condition. Differences between the first constraint condition, the second constraint condition, and the third constraint condition are specifically described below.

The processing module is configured to perform processing based on the constraint condition sent by the terminal, to obtain a new molecule. Specific processing is as follows.

The molecule generation submodule is configured to generate a molecule based on a generation parameter. The molecule generation submodule has a built-in molecule database, and the molecule database stores a plurality of different molecules. Optionally, the molecule database may store molecular structures or molecular formulas of the molecules, or may include molecular structures and molecular formulas of the molecules. A molecule generation rule is set in the molecule generation submodule, and the molecule generation submodule may generate a plurality of new molecules based on the molecule database and the molecule generation rule. Optionally, in a possible implementation, the molecule generation submodule stores a molecule generation model, and the molecule generation submodule may generate a plurality of new molecules based on the molecule database and the molecule generation model. The molecule generation model may be obtained through training based on another molecule database and another new molecule. Optionally, the molecule generation rule may be modeled, that is, the molecule generation rule is used to generate a model, to obtain the molecule generation model. The molecule generation model may be obtained through supervised training or unsupervised training. This is not limited in this application.

Optionally, the generation parameter may be set by the user via the terminal, or the generation parameter may be randomly generated by the server.

Optionally, the molecule database in the molecule generation submodule may be provided by a customer. The customer may provide a corresponding molecule database based on a specific requirement, and provide different molecule databases for different application scenarios and different requirements.

The molecular property determining submodule is configured to determine a property of a molecule.

Optionally, the molecular property determining submodule may be configured to determine the property of the molecule based on a molecular structure or a molecular formula. The molecular structure includes some substructures. Some specific substructures have some fixed properties. For example, a chemical bond has one or more specific properties, and an atom or a group has one or more specific properties. If the molecule includes these specific substructures, the molecule has the properties of the specific substructures. Alternatively, based on the molecular formula, it may be known which specific groups are included in the molecule, and the molecule has properties of the specific groups.

Optionally, the molecular property determining submodule may be further configured to predict the property of the molecule based on a molecular property prediction model. The molecular property prediction model is obtained through training based on a large quantity of molecules and property information of each of these molecules, where the molecule may exist in a form of a molecular structure, or may exist in a form of a molecular formula. These molecules may be molecules in the molecule database in the foregoing molecule generation submodule, or may be other molecules. Optionally, the molecular property prediction model may be provided by the customer. To be specific, the customer uploads, to the server via the terminal, the molecular property prediction model trained by the customer, to perform property prediction on the molecules.

In this application, the molecular property determining submodule is configured to provide property information for each of the plurality of molecules generated by using the molecule generation submodule.

The molecule filtering submodule is configured to filter out some molecules based on the second constraint condition, to obtain a molecule that meets the constraint condition, where the second constraint condition indicates a condition that a property of a molecule needs to meet. When a molecule meets the second constraint condition, the molecule is retained, or when a molecule does not meet the second constraint condition, the molecule is discarded. The second constraint condition may include one constraint condition, or may include a plurality of constraint conditions. This is not specifically limited in this application. During actual application, the second constraint condition may be specifically set based on the specific requirement. It should be noted that, for ease of understanding, nouns herein are consistent with those in the claims. The "second constraint condition" appears first, where the second constraint condition is used to filter and screen molecules. The "first constraint condition" and the "third constraint condition" appear later, where the "first constraint condition" or the "third constraint condition" is used to score the molecules.

For example, the second constraint condition may be that a molecular weight is in a range greater than or equal to 500 and less than or equal to 600. If a molecular weight of a molecule is in the range greater than or equal to 500 and less than or equal to 600, the molecule meets the second constraint condition, and the molecule is retained. If a molecular weight of a molecule is less than 500 or greater than 600, the molecule does not meet the second constraint condition, and the molecule is discarded. The second constraint condition may further constrain one or more of water solubility, lipid solubility, bioactivity, synthesizability, a docking binding affinity for a specific molecule, a similarity to a source molecule, and the like of the molecule. For example, the second constraint condition may constrain one or more of a water solubility range, a lipid solubility range, a bioactivity range, a synthesizability value, a value of a docking binding affinity for a specific molecule, a value of a similarity to a source molecule of the molecule, and the like.

When the second constraint condition includes one constraint condition, a molecule can be retained as long as the molecule meets the constraint condition in the second constraint condition; otherwise, the molecule is discarded. When the second constraint condition includes a plurality of constraint conditions, and the plurality of constraint conditions are in an "and" relationship, a molecule can be retained only when the molecule meets the plurality of constraint conditions in the second constraint condition at the same time; otherwise, the molecule is discarded. When the second constraint condition includes a plurality of constraint conditions, and the plurality of constraint conditions are in an "or" relationship, a molecule can be retained only when the molecule meets at least one of the plurality of constraint conditions; or when a molecule meets none of the plurality of constraint conditions, the molecule is discarded, and the like.

In this application, the molecule filtering submodule is configured to filter, based on the second constraint condition, the plurality of molecules generated by the molecule generation submodule, to be specific, determine whether a property of each of the plurality of molecules generated by the molecule generation submodule meets the second constraint condition, and if the property meets the second constraint condition, retain the molecule, or if the property does not meet the second constraint condition, discard the molecule.

The molecule scoring submodule is configured to score a molecule based on the first constraint condition or the third constraint condition. Herein, the first constraint condition is used as an example for description. The third constraint condition is similar to the first constraint condition. In this application, the first constraint condition and the third constraint condition are merely different in names in the following different method embodiments. Actually, the first constraint condition and the third constraint condition may be the same or may be different. The first constraint condition indicates a condition that a property of a molecule needs to meet. The first constraint condition may include one constraint condition, or may include a plurality of constraint conditions. For example, the first constraint condition may include that a molecular weight of the molecule is in a range greater than or equal to 500 and less than or equal to 600, the first constraint condition may further include that the molecule is water-soluble, the first constraint condition may further include that a similarity to a source molecule is greater than 60%, and the like.

For ease of understanding, an example in which the first constraint condition includes three constraint conditions is used for explanation and description. The first constraint condition includes: (i) a molecular weight is greater than or equal to 500 and less than or equal to 600; (ii) the molecule is water-soluble; and (iii) a similarity to a source molecule is greater than 60%. It is assumed that, if the molecule meets the constraint condition (i), the molecule is scored a, or if the molecule does not meet the constraint condition (i), the molecule is scored 0; if the molecule meets the constraint condition (ii), the molecule is scored b, or if the molecule does not meet the constraint condition (ii), the molecule is scored 0; or if the molecule meets the constraint condition (iii), the molecule is scored c, or if the molecule does not meet the constraint condition (iii), the molecule is scored 0. Values of a, b, and c may be equal. For example, a, b, and c are all 1, to be specific, each time the molecule meets one of the constraint conditions, 1 point is obtained. Alternatively, values of a, b, and c may be unequal. For example, a is 1, b is 2, and c is 3, to be specific, if the molecule meets the constraint condition (i), 1 point is obtained; if the molecule meets the constraint condition (ii), 2 points are obtained; and if the molecule meets the constraint condition (iii), 3 points are obtained. For another example, a is 1, b is 2, and c is 2. Details are not described herein again.

In an example, for each molecule, a scoring result may be obtained by directly adding scores of constraint conditions. For example, if a molecule meets the constraint conditions (i), (ii), and (iii) at the same time, the molecule is scored (a+b+c), if a molecule meets the constraint conditions (i) and (ii) at the same time, the molecule is scored (a+b), if a molecule meets the constraint conditions (ii) and (iii), the molecule is scored (b+c), or the like.

In an example, for each molecule, a scoring result may be obtained by weighting scores of constraint conditions. For example, the constraint condition (i) is weighted 40%, the constraint condition (ii) is weighted 20%, and the constraint condition (iii) is weighted 40%. In this case, if a molecule meets the constraint conditions (i), (ii), and (iii) at the same time, the molecule is scored (a*40%+b*20%+c*40%), if a molecule meets the constraint conditions (i) and (ii), the molecule is scored (a*40%+b*20%), if a molecule meets the constraint conditions (ii) and (iii), the molecule is scored (b*20%+c*40%), or the like. During specific implementation, a weight of each constraint condition may be specifically set based on a specific case. Usually, a more important property indicates a larger weight of a constraint condition to which the property belongs, or indicates a higher score (a, b, or c) that is set for a constraint condition to which the property belongs.

In an example, constraint conditions and scores may alternatively be as follows: (i) if a molecular weight is greater than or equal to 400 and less than 500, a molecule is scored a; if a molecular weight is greater than or equal to 500 and less than 600, a molecule is scored b; if a molecular weight is greater than or equal to 600 and less than 700, a molecule is scored c; and in other cases, a molecule is scored 0; (ii) if a similarity to a source molecule is greater than or equal to 50% and less than 70%, a molecule is scored d; if a similarity to a source molecule is greater than or equal to 70% and less than 85%, a molecule is scored e; if a similarity to a source molecule is greater than or equal to 85% and less than 90%, a molecule is scored f; if a similarity to a source molecule is greater than or equal to 90%, a molecule is scored g; and in other cases, a molecule is scored 0; and (iii) if a molecule is water-soluble, the molecule is scored h; or if a molecule is water-insoluble, the molecule is scored 0. If a molecular weight of a molecule is 566, a similarity to a source molecule is 75%, and the molecule is water-soluble, a scoring result of the molecule is (b+e+h). If a molecular weight of a molecule is 578, a similarity to a source molecule is 85%, and the molecule is water-insoluble, a scoring result of the molecule is (b+f).

In the foregoing examples, the first constraint condition is merely used as an example. During actual application, more or fewer constraint conditions may be set based on a specific application scenario and a specific requirement. The scoring rules in the foregoing examples are merely used as examples. The scoring rule is not specifically limited in this application. During actual application, the scoring rule may be specifically set by the user based on a specific case.

The first constraint condition may be an affirmative sentence representing a requirement, for example, a molecular weight is greater than or equal to 500 and less than or equal to 600, for another example, a molecule is water-soluble, or for another example, a similarity to a source molecule is greater than 60%. Alternatively, the first constraint condition may be an interrogative sentence. For example, the constraint conditions (i), (ii), and (iii) in the foregoing examples may be respectively represented by interrogative sentences as follows: (i) whether a molecular weight is in a range greater than or equal to 500 and less than or equal to 600; (ii) whether a molecule is water-soluble; and (iii) whether a similarity to a source molecule is greater than 60%. In a case of representation by using an interrogative sentence, for a molecule, if an answer is "yes", it indicates that the constraint condition is met; and if the answer is "no", it indicates that the constraint condition is not met. For example, for the constraint condition (i), if the answer is "yes", it indicates that the constraint condition (i) is met, if the answer is "no", it indicates that the constraint condition (i) is not met. The same applies to the constraint conditions (ii) and (iii). Alternatively, the constraint condition may be expressed in another form. A specific expression form of the constraint condition is not limited in this application. Similarly, an expression form of the foregoing second constraint condition is not specifically limited in this application.

In this application, the molecule scoring submodule is configured to score, based on the first constraint condition or the third constraint condition, one or more molecules retained after filtering by the molecule filtering submodule.

After a scoring result of each molecule is obtained, the scoring result of each molecule is fed back to the molecule generation submodule. Specifically, the scoring result of each molecule affects a generation parameter in the molecule generation submodule. In other words, the generation parameter is obtained through calculation based on the scoring result of each molecule, and there is a mapping relationship between the generation parameter and the scoring result of each molecule. To be specific, molecules may be optimized through a plurality of iterations. Molecules obtained in each iteration are scored, a generation parameter is adjusted based on scoring results, new molecules are generated based on an adjusted generation parameter, properties of the new molecules are determined, the molecules are filtered based on the properties of the molecules, molecules that are retained after the filtering are scored, and the generated parameter is adjusted based on scoring results again, ..., to optimize the molecules and obtain a molecule that meets a requirement. A specific procedure and method for generating and optimizing molecules are described below. For details, refer to descriptions in the following method embodiments.

Optionally, in a first iteration, a generation parameter may be randomly generated by the server, or may be set by the user. In a second iteration and subsequent iterations, the generation parameter may be adjusted based on a scoring result in a previous iteration.

In the server shown in FIG. 2, the molecule filtering submodule is optional. In other words, the server includes the molecule generation submodule, the molecular property determining submodule, and the molecule scoring submodule. The molecule generation submodule is configured to generate molecules based on the generation parameter, the molecular property determining submodule is configured to provide property information for the molecules generated by the molecule generation submodule, the molecule scoring submodule is configured to score, based on the first constraint condition, each molecule generated by the molecule generation submodule, a scoring result of each molecule is applied to the molecule generation submodule, a generation parameter in the molecule generation submodule is adjusted based on the scoring result of each molecule, the molecule generation submodule generates molecules again based on an adjusted generation parameter, the molecular property determining submodule determines property information of each generated molecule again, and the molecule scoring submodule scores each generated molecule again, ..., to optimize the molecules and obtain a molecule that meets a requirement.

Optionally, the server provides one or more open application programming interfaces (application programming interfaces, APIs). The user may upload data, a model, a molecule database, or the like to the server through the one or more APIs. For example, the user may upload the molecule database to the server through the APIs, so that the molecule generation submodule generates molecules based on the molecule database uploaded by the user. For another example, the user may upload, to the server through the APIs, a molecular property prediction model trained by the user, so that the molecular property determining submodule predicts a property of a molecule based on the molecular property prediction model uploaded by the user; or the user uploads, to the server through the APIs, a molecule designed by the user, and provides property information for the molecule uploaded by the user, without the need for the molecule generation submodule to generate the molecule, and the like.

Optionally, the server is located on the cloud, and scalability is relatively good. A functional component may be added to or deleted from the server based on a specific requirement, to obtain more functions related to molecule generation through extension.

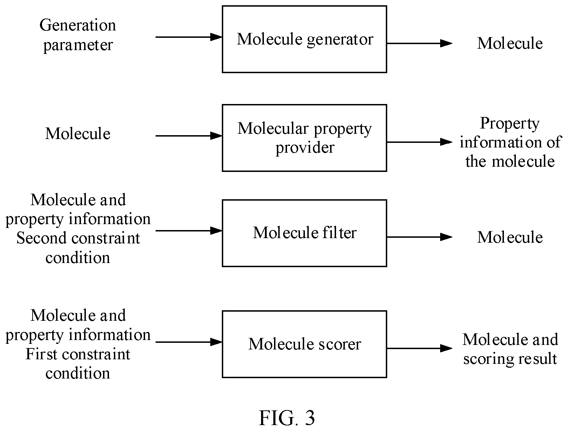

Each component in the server in FIG. 2 may run independently. Refer to a diagram shown in FIG. 3. When the molecule generation submodule runs independently, an input is a generation parameter, and an output is a molecule. The generation parameter can be configured by the user. One or more molecules may be output. When the molecular property determining submodule runs independently, an input is a molecule, and an output is property information of the molecule. One or more molecules may be input. A form of the input molecule may include a molecular structure or a molecular formula, or may include both a molecular structure and a molecular formula. When the input is one molecule, the output includes property information of the one molecule, or when the input includes a plurality of molecules, the output includes property information of each of the plurality of molecules. When the molecule filtering submodule runs independently, an input is a second constraint condition, one or more molecules, and corresponding property information, and an output is one or more molecules that meet the second constraint condition, where the second constraint condition includes one or more constraint conditions. When the molecule scoring submodule runs independently, an input is a first constraint condition, one or more molecules, and corresponding property information, and an output is one or more molecules and corresponding scoring results, where the first constraint condition includes one or more constraint conditions. The user may input data on the terminal. The server obtains the input data input by the user through an API, performs corresponding processing by using a component, obtains corresponding output data, and returns the output data to the terminal.

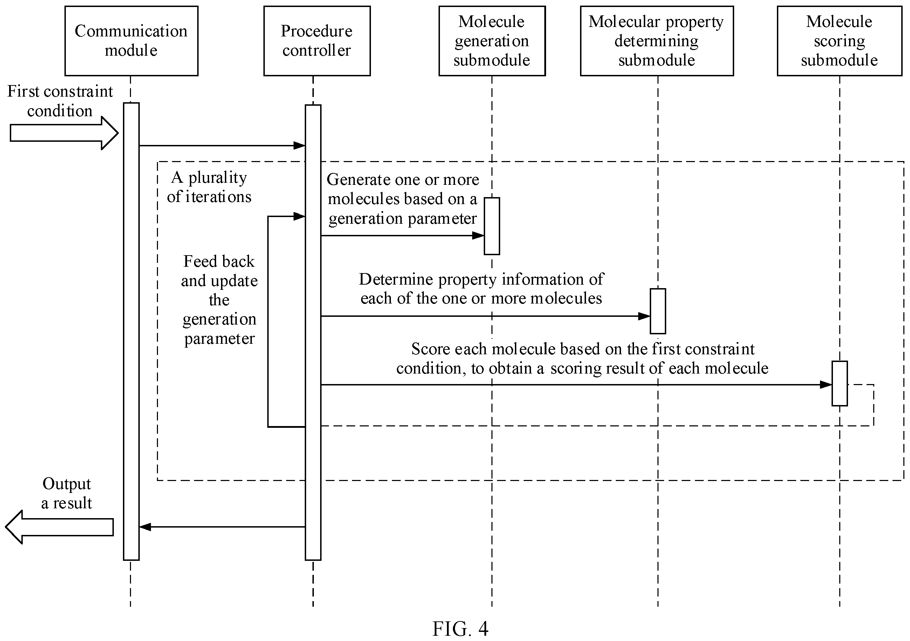

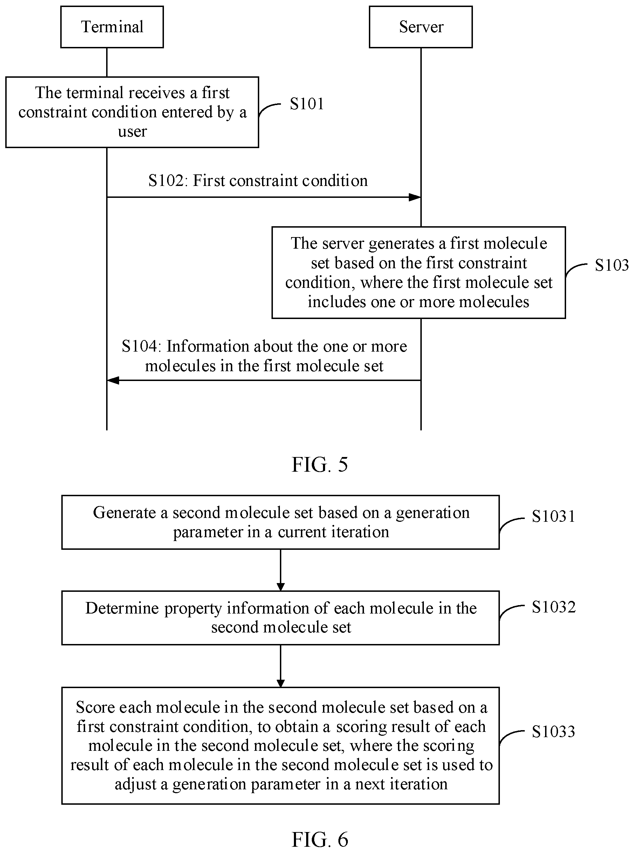

This application provides a molecule generation method. The molecule generation method is applied to a server, and the server includes a communication module, a molecule generation submodule, a molecular property determining submodule, and a molecule scoring submodule. FIG. 4 is a diagram of a molecule generation time sequence on a server side according to this application. A procedure controller is added in FIG. 4. The procedure controller is configured to control an execution process of a procedure. During actual implementation, the procedure controller may be virtual or may not exist. Herein, the procedure controller is merely for ease of understanding and drawing. FIG. 5 is a schematic flowchart of a molecule generation method according to this application. The following describes the molecule generation method provided in embodiments with reference to FIG. 4 and FIG. 5. The method includes but is not limited to descriptions of the following content.

S101: A terminal receives a first constraint condition entered by a user.

The terminal may be a mobile phone, a personal laptop computer, a desktop computer, or the like. For the first constraint condition, refer to the descriptions of the related content in FIG. 2. For brevity of the specification, details are not described herein again.

S102: The terminal sends, to the server, the first constraint condition entered by the user, and correspondingly, the server receives the first constraint condition entered by the user on the terminal.

S103: The server generates a first molecule set based on the first constraint condition, where the first molecule set includes one or more molecules.

That the server generates a first molecule set based on the first constraint condition is implemented through a plurality of iterations. The following describes how the plurality of iterations are implemented. FIG. 6 is a diagram of a partial procedure of a molecule generation method according to this application. The method includes but is not limited to descriptions of content of steps S1031 to S1033.

S1031: Generate a second molecule set based on a generation parameter in a current iteration.

Refer to FIG. 4. In this embodiment, this step may be performed by the molecule generation submodule. The molecule generation submodule generates one or more molecules based on the generation parameter. For ease of description, the one or more molecules generated by the molecule generation submodule herein are referred to as the second molecule set, or the second molecule set includes the one or more molecules generated by the molecule generation submodule.

The current iteration may be a first iteration, or may be a second iteration or any iteration after the second iteration. For the first iteration, the generation parameter may be randomly generated by the server, or may be configured by the user. For the second iteration or any iteration after the second iteration, the generation parameter is determined based on scoring results of one or more molecules obtained in a previous iteration. For example, in the second iteration, the generation parameter is determined based on a scoring result of each molecule generated by the molecule generation submodule in the first iteration; and in a third iteration, the generation parameter is determined based on a scoring result of each molecule generated by the molecule generation submodule in the second iteration.

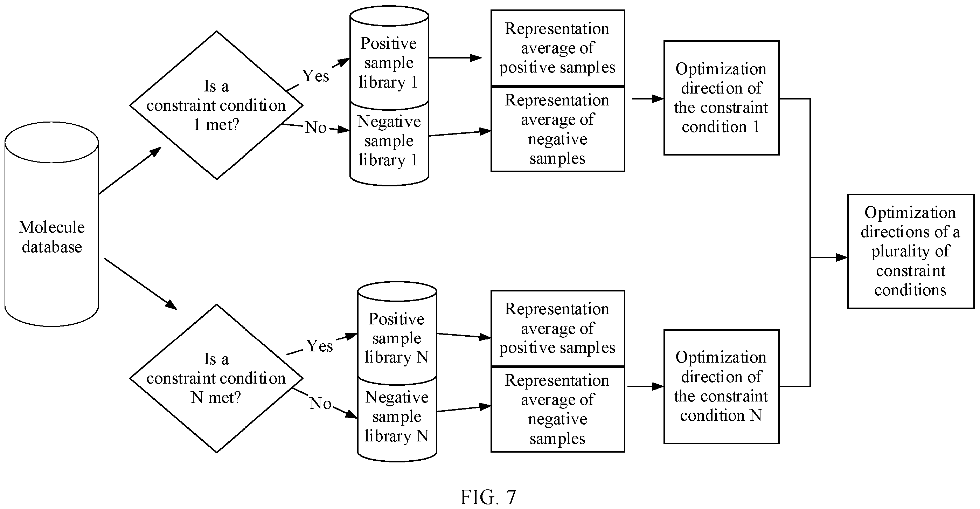

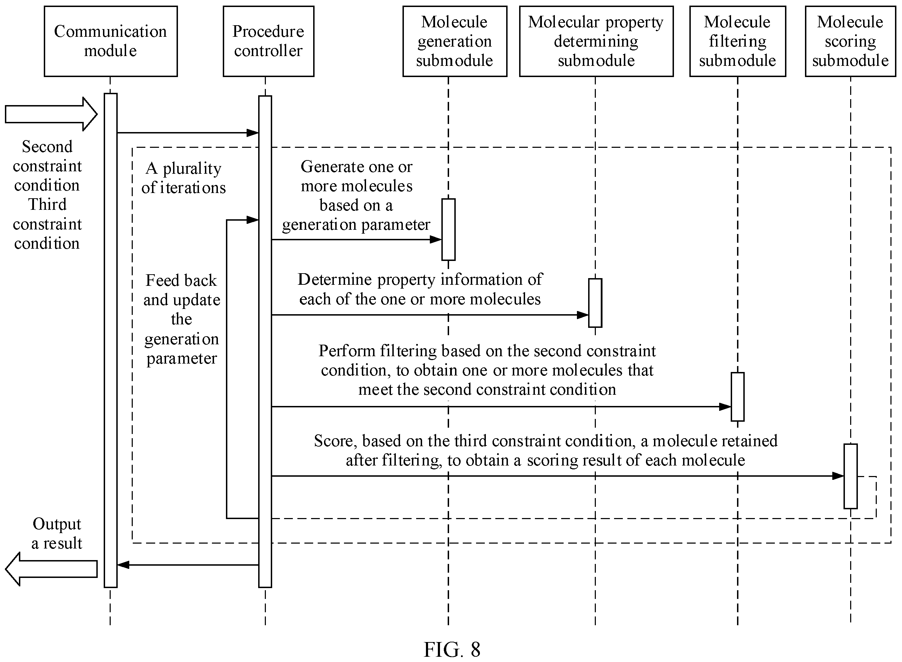

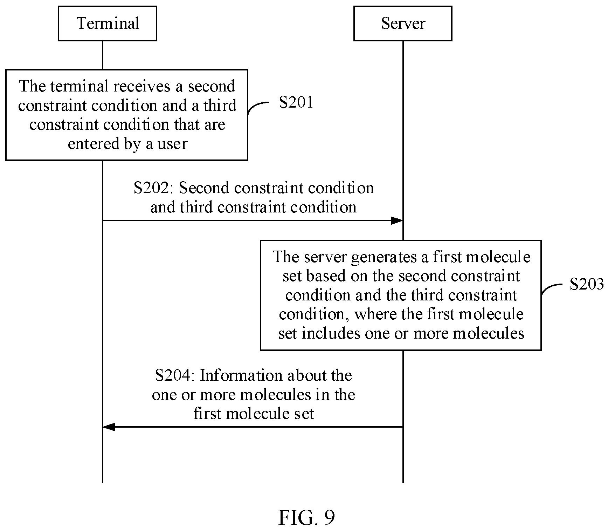

Optionally, the server or the molecule generation submodule in the server may alternatively generate a molecule by using a deep learning method. When the molecule generation submodule generates a molecule by using the deep learning method, if the current iteration is the first iteration, this application provides a method for generating a molecule based on a molecule database. The method is summarized as follows.
(1) Property information of each molecule in the molecule database is determined by using a molecular property determining submodule in the server, where each molecule in the molecule database is stored in a form of a molecular structure.
(2) It is assumed that the user enters a plurality of constraint conditions, and the plurality of constraint conditions include a constraint condition 1, ..., and a constraint condition N. For each constraint condition k, it is determined whether each molecule in the molecule database meets the constraint condition k, a molecule that meets the constraint condition k is classified into a positive sample library k, and a molecule that does not meet the constraint condition k is classified into a negative sample library k. For example, refer to a diagram shown in FIG. 7. For the constraint condition 1, it is determined whether each molecule in the molecule database meets the constraint condition 1, and if a molecule in the molecule database meets the constraint condition 1, the molecule is classified into a positive sample library 1, or if a molecule in the molecule database does not the constraint condition 1, the molecule is classified into a negative sample library 1, ..., and for the constraint condition N, it is determined whether each molecule in the molecule database meets the constraint condition N, and if a molecule in the molecule database meets the constraint condition N, the molecule is classified into a positive sample library N, or if a molecule in the molecule database does not meet the constraint condition N, the molecule is classified into a negative sample library N.
   It should be noted that, for this embodiment, the plurality of constraint conditions entered by the user may include a part or all of the first constraint condition in step S1033 in this embodiment, or may include another condition related to the property information other than the first constraint condition in this embodiment. This is not limited in this application. For the following embodiment shown in FIG. 8 or FIG. 9, the plurality of constraint conditions entered by the user may include a part or all of the second constraint condition in step S2034, or may include a part or all of the third constraint condition in step S2033, or may include another condition related to the property information other than the second constraint condition and the third constraint condition in the embodiment in FIG. 8 or FIG. 9. This is not limited in this application.
(3) A representation average of all samples in each sample library is calculated. For example, a representation average of all positive samples in the positive sample library 1 is calculated, a representation average of all negative samples in the negative sample library 1 is calculated, ..., a representation average of all positive samples in the positive sample library N is calculated, and a representation average of all negative samples in the negative sample library N is calculated. Each molecule has a representation form in high-dimensional space. A representation average of all samples in a sample library can be obtained by calculating an average of representations of all samples in the sample library in the high-dimensional space.
(4) An optimization direction of each constraint condition is determined based on the representation average of all samples in each sample library.

For the constraint condition 1, an optimization direction of the constraint condition 1 is determined based on the representation average of all samples in the positive sample library 1 and the representation average of all samples in the negative sample library 1. Similarly, for the constraint condition N, an optimization direction of the constraint condition N is determined based on the representation average of all samples in the positive sample library N and the representation average of all samples in the negative sample library N. In a possible implementation, the optimization direction of the constraint condition 1 is obtained by subtracting the representation average of all samples in the negative sample library 1 from the representation average of all samples in the positive sample library 1. Similarly, the optimization direction of the constraint condition N is obtained by subtracting the representation average of all samples in the negative sample library N from the representation average of all samples in the positive sample library N.

It may be understood that the optimization direction of each constraint condition represents an optimization direction of each molecule in terms of a molecular property corresponding to the constraint condition.

(5) An optimization direction of the plurality of constraint conditions is determined based on the optimization direction of each constraint condition.

In an embodiment, one optimization direction of the plurality of constraint conditions may be obtained by directly adding the optimization directions of all constraint conditions. In another embodiment, weighted summation is performed on the optimization directions of all constraint conditions, to obtain the optimization direction of the plurality of constraint conditions, where weights may be randomly generated, or may be set by the user. For example, a weight of the optimization direction of the constraint condition 1 is 20%, a weight of the optimization direction of the constraint condition N is 10%, and weights of optimization directions of other constraint conditions are 70%/(N-2). A plurality of optimization directions of the plurality of constraint conditions may be obtained by performing weighted summation for a plurality of times. Therefore, one optimization direction of the plurality of constraint conditions or a plurality of optimization directions of the plurality of constraint conditions may be obtained based on the optimization direction of each constraint condition.

In deep learning, one optimization direction of the plurality of constraint conditions or a plurality of optimization directions of the plurality of constraint conditions may be used as a generation parameter to be input to the molecule generation submodule. The molecule generation submodule generates one or more molecules based on the one optimization direction of the plurality of constraint conditions or the plurality of optimization directions of the plurality of constraint conditions, that is, obtains the second molecule set.

In the first iteration, the second molecule set is obtained by using this method, which lays a good foundation for molecule optimization, can efficiently obtain a molecule that meets a requirement, and improves optimization efficiency.

S1032: Determine property information of each molecule in the second molecule set.

Refer to FIG. 4. In this embodiment, this step may be performed by the molecular property determining submodule. The molecular property determining submodule determines property information of each of the one or more molecules generated by the molecule generation submodule. For the property information of the molecule, refer to the related descriptions of the property information of the molecule in FIG. 2. For brevity of the specification, details are not described herein again. During actual application, which property information of the molecule specifically needs to be determined may be set based on a specific requirement. This is not limited in this application.

The molecular property determining submodule may determine a property of a molecule based on a molecular structure or a molecular formula, or may perform prediction based on a molecular property prediction model. For descriptions of the molecular property prediction model, refer to the descriptions of the related content in FIG. 2. Details are not described herein again. In addition, the molecular property prediction model may be trained based on a graph encoder, where a graph includes a molecular structure, and the training may use a supervised training method or an unsupervised training method. The training method for the molecular property prediction model is not limited in this application.

S1033: Score each molecule in the second molecule set based on the first constraint condition, to obtain a scoring result of each molecule in the second molecule set, where the scoring result of each molecule in the second molecule set is used to adjust a generation parameter in a next iteration.

Refer to FIG. 4. In this embodiment, this step may be performed by the molecule scoring submodule. After the property information of each of the one or more molecules generated by the molecule generation submodule is determined, the molecule scoring submodule scores the one or more molecules based on the first constraint condition, to obtain the scoring result of each molecule. For related content such as the first constraint condition and the scoring rule, refer to the descriptions of the related content in FIG. 2. Details are not described herein again.

As shown in FIG. 4, scoring results of the one or more molecules generated by the molecule generation submodule in the current iteration are used to adjust the generation parameter in the next iteration. Optionally, in a possible implementation, there is a mapping relationship between the scoring result of each molecule in the current iteration and the generation parameter in the next iteration. The mapping relationship may be reflected by using a function, a formula, or the like, or may be reflected by using a list. The scoring result of each molecule in the list may alternatively be replaced with "an average of scoring results of the molecules".

In a possible implementation, alternatively, there may be a difference between an average of scoring results of the molecules in the current iteration and an average of scoring results of the molecules in a previous iteration. The difference may be a positive number, a negative number, or 0. A molecule generation system stores a relationship between the difference and an adjustment direction of the generation parameter. The relationship may be reflected by using a function or a formula, or may be reflected by using a list. The difference may be a difference between averages of scoring results in two iterations, or may be a difference between sums of scoring results in two iterations, or may be a difference between variances of scoring results in two iterations, or the like. The difference may be replaced with a "ratio".

A maximum number of iterations may be set in the server. When the maximum number of iterations is reached, the iteration ends. Alternatively, a condition for ending the iteration may be set based on scoring results. For example, it may be set that the iteration ends when an average of scoring results of the molecules reaches a first threshold, or it is set that the iteration ends when a scoring result of each molecule is greater than or equal to a second threshold, or the like. The first threshold or the second threshold may be specifically set by the user based on an actual case. The condition for ending the iteration may alternatively be another condition. For example, a quantity of obtained molecules may be constrained as the condition for ending the iteration, or a similarity between each molecule and a source molecule may be constrained as the condition for ending the iteration, or other property information of the molecule may be constrained as the condition for ending the iteration, or the like. The condition for ending the iteration is not limited in this application.

Optionally, an output result may be one or more molecules obtained in each iteration and a scoring result corresponding to each molecule, that is, one or more molecules obtained in a current iteration and a scoring result corresponding to each molecule are output in each iteration. Alternatively, an output result may be one or more molecules obtained in a last iteration and a scoring result corresponding to each molecule. Optionally, the output result may alternatively not include the scoring result of each molecule. A final output result may be set based on a specific case. This is not limited in this application.

Optionally, when the output result includes a plurality of molecules and a scoring result of each of the plurality of molecules, the molecule generation system may perform sorting based on the scoring result of each molecule.

Optionally, when the output result includes the plurality of molecules, the server may further sort the molecules based on strength or a size of a specific property, for example, may sort the molecules in ascending or descending order of molecular weights, or may sort the molecules in descending or ascending order of water solubility, or may sort the molecules in ascending or descending order of similarities to the source molecule, or the like.

S104: The server returns information about the one or more molecules in the first molecule set to the terminal.

The server returns the output result to the terminal. Correspondingly, the terminal receives the output result sent by the server, where the output result includes the information about the one or more molecules in the first molecule set.

This application further provides a molecule generation method. The method may be applied to the server shown in FIG. 1, and the server includes a communication module, a molecule generation submodule, a molecular property determining submodule, a molecule filtering submodule, and a molecule scoring submodule, as shown in FIG. 8. FIG. 8 is a diagram of a molecule generation time sequence on a server side according to an embodiment. A procedure controller is added in FIG. 8. The procedure controller is configured to control an execution process of a procedure. During actual implementation, the procedure controller may be virtual or may not exist. Herein, the procedure controller is merely for ease of understanding and drawing. FIG. 9 is a schematic flowchart of another molecule generation method according to this application. The method includes but is not limited to descriptions of the following content.

S201: A terminal receives a second constraint condition and a third constraint condition that are entered by a user.

S202: The terminal sends, to a server, the second constraint condition and the third constraint condition that are entered by the user, and correspondingly, the server receives the second constraint condition and the third constraint condition that are entered by the user on the terminal.

S203: The server generates a first molecule set based on the second constraint condition and the third constraint condition, where the first molecule set includes one or more molecules.

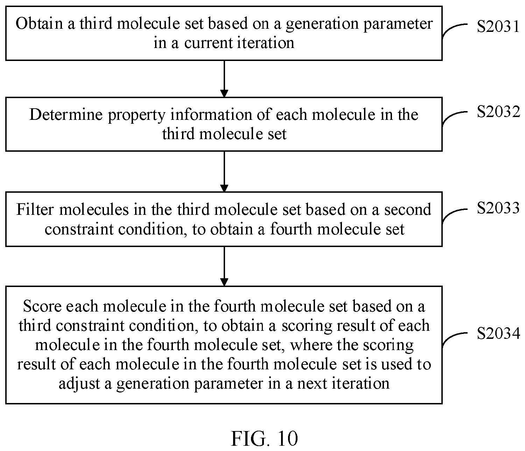

That the server generates the first molecule set based on the second constraint condition and the third constraint condition is implemented through a plurality of iterations. The following describes a process of the plurality of iterations. FIG. 10 is a schematic flowchart of a molecule generation method on a server side according to this application. The method includes but is not limited to descriptions of content of steps S2031 to S2034.

S2031: Obtain a third molecule set based on a generation parameter in a current iteration.

S2032: Determine property information of each molecule in the third molecule set.

S2033: Filter molecules in the third molecule set based on the second constraint condition, to obtain a fourth molecule set.

S2034: Score each molecule in the fourth molecule set based on the third constraint condition, to obtain a scoring result of each molecule in the fourth molecule set, where the scoring result of each molecule in the fourth molecule set is used to adjust a generation parameter in a next iteration.

As shown in FIG. 8, in this embodiment, step S2031 may be performed by the molecule generation submodule in the server. The third molecule set is used in step S2031 to keep consistent with a noun in the claims, and the third molecule set includes one or more molecules. Step S2032 may be performed by the molecular property determining submodule in the server, and the molecular property determining submodule in a generation system determines property information of each molecule in the third molecule set.

A difference between this embodiment and the method embodiment shown in FIG. 4 or FIG. 5 lies in that step S2033 is added in this embodiment. It can be learned from FIG. 8 that step S2033 may be performed by the molecule filtering submodule in the server. After the property information of each molecule in the third molecule set is determined, the molecule filtering submodule filters the molecules in the third molecule set based on the second constraint condition, to obtain the fourth molecule set that meets the second constraint condition, where the fourth molecule set may include one molecule or may include a plurality of molecule. For the second constraint condition, refer to the descriptions of the related content in FIG. 2. Details are not described herein again. Step S2034 may be performed by the molecule scoring submodule in the server. The molecule scoring submodule scores each molecule in the fourth molecule set based on the third constraint condition, to obtain the scoring result of each molecule in the fourth molecule set. The scoring result of each molecule in the fourth molecule set is used to adjust the generation parameter in the next iteration.

Optionally, the first molecule set may include a molecule in a fourth molecule set obtained in each iteration, or the first molecule set is a molecule in a fourth molecule set obtained in a last iteration. For a final output result of the server, refer to the description of the content of S1033.

S204: The server returns information about the one or more molecules in the first molecule set to the terminal.

For the content of steps S2031, S2032, and S2034, refer to the descriptions of step S1031 to step S1033 in the method embodiment in FIG. 4 or FIG. 5. For step S2033, refer to the descriptions of the related content in FIG. 2. For brevity of the specification, details are not described herein again.

It can be learned that this application provides a molecule generation method. When molecules are to be generated, the user only needs to enter a constraint condition, and a system generates new molecules based on the constraint condition, and optimizes the new molecules through a plurality of iterations, to finally obtain a new molecule that meets the constraint condition. The molecule generation method provided in this application is used to generate and optimize molecules, which has advantages of convenient operation, high efficiency, and cost reduction. The method provided in this application is applicable to molecule generation in any scenario. In addition, each component in the server may run independently, to implement a function corresponding to each component. For example, the molecule generation submodule may be configured to generate a new molecule based on a molecule database; the molecular property determining submodule may be configured to provide property information for an input molecule; the molecule filtering submodule may be configured to filter a plurality of input molecules; and the molecule scoring submodule may be configured to score the input molecules.

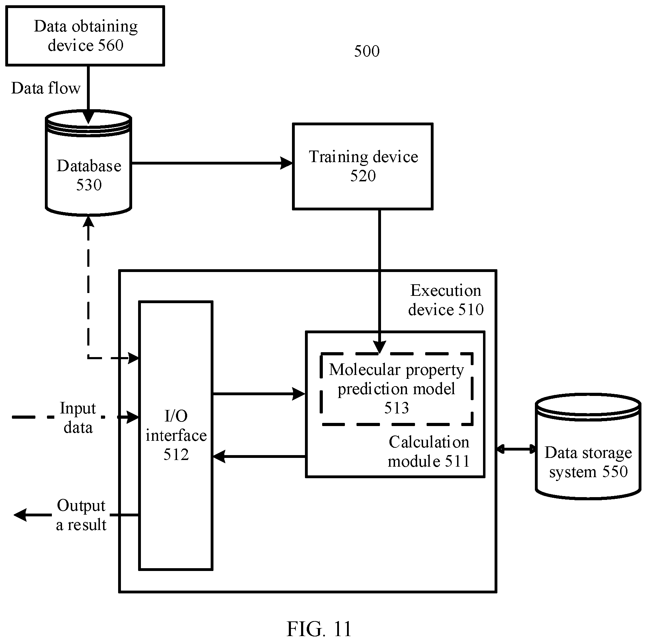

FIG. 11 is a diagram of training and prediction structures of a molecular property prediction model 513 according to an embodiment of this application. As shown in FIG. 11, a data obtaining device 560 is configured to obtain training data, where the training data includes a plurality of molecules and property information of each of the plurality of molecules, and the molecule may exist in a form of a molecular structure, or may exist in a form of a molecular formula. This is not limited herein.

After obtaining the training data, the data obtaining device 560 stores the training data in a database 530, and the database 530 may maintain the training data. A training device 520 may perform training based on the training data in the database 530, to obtain a trained molecular property prediction model 513, and transplant the trained molecular property prediction model 513 to an execution device 510. Optionally, the training device 520 may exist independently of the execution device 510, or may be integrated inside the execution device 510.

A user may enter, through an input/output I/O interface 512 of the execution device 510, a plurality of molecules whose properties need to be predicted, or may store, via the data obtaining device 560 into the database 530, a plurality of molecules whose properties need to be predicted, and then, the execution device 510 obtains, from the database 530, the plurality of molecules whose properties need to be predicted. The molecular property prediction model 513 performs property prediction on the plurality of entered molecules, obtains property information of each molecule, and outputs each molecule and corresponding property information through the input/output I/O interface 512.

It should be noted that, during actual application, the training data maintained in the database 530 is not necessarily from the data obtaining device 560, and may also be obtained from another device. In addition, it should be noted that the training device 520 does not necessarily train the molecular property prediction model 513 completely based on the training data maintained in the database 530, or may obtain training data from another device to perform model training. The foregoing description should not be used as a limitation on this embodiment of this application.

The molecular property prediction model 513 may be used in the server shown in FIG. 2 in this application, and specifically, applied to the molecular property determining submodule in the server. When the molecular property prediction model 513 is configured to perform molecular property prediction, the molecular property prediction model 513 may also be used as an independent component, for example, the molecular property determining submodule in FIG. 3. When the molecular property prediction model 513 is used in the server shown in FIG. 2, the input/output I/O interface 512 may be the API in the server, that is, the user enters, into the server through the API, the plurality of molecules whose properties need to be predicted.

When the execution device 510 processes input data, or when a calculation module 511 of the execution device 510 performs calculation-related processing, the execution device 510 may invoke data, code, and the like in a data storage system 550 for corresponding processing, or may store data, instructions, and the like obtained through corresponding processing into the data storage system 550.

It should be noted that the training device 520 may generate corresponding molecular property prediction models 513 for different targets based on different training data. The corresponding molecular property prediction models 513 may be used to implement the foregoing targets, to provide a required result for the user.

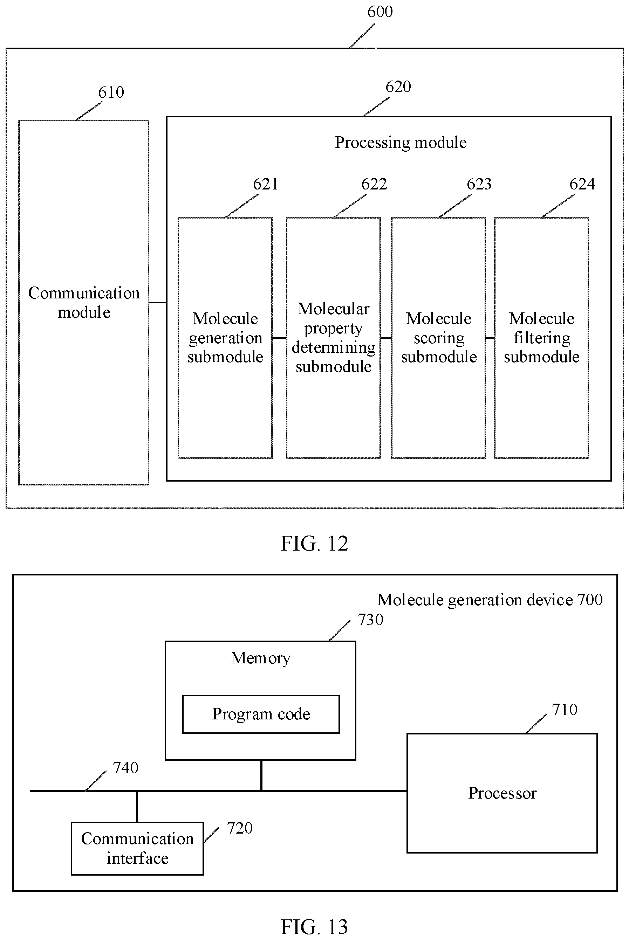

This application provides a molecule generation apparatus 600. The molecule generation apparatus 600 may be the server in FIG. 1 or FIG. 2. FIG. 12 is a diagram of a structure of the molecule generation apparatus 600 according to this application. The molecule generation apparatus 600 includes the following modules.

A communication module 610 is configured to receive a constraint condition entered by a user on a terminal, where the constraint condition indicates a condition that a property of a molecule needs to meet, and the property of the molecule includes any one or more of a molecular weight, water solubility, lipid solubility, bioactivity, synthesizability, a docking binding affinity for a specific molecule, a similarity to a source molecule, an included specific substructure, pharmacokinetics, and toxicity of the molecule. A processing module 620 is configured to generate a first molecule set based on the constraint condition, where the first molecule set includes one or more molecules. The communication module 610 is further configured to return information about the one or more molecules in the first molecule set to the terminal.

In a possible implementation, the processing module 620 includes a molecule generation submodule 621, a molecular property determining submodule 622, and a molecule scoring submodule 623. The constraint condition includes a first constraint condition, and the generating a first molecule set based on the constraint condition includes a plurality of iterations. In each iteration,
the molecule generation submodule 621 is configured to generate a second molecule set based on a current generation parameter, where the second molecule set includes one or more molecules;
the molecular property determining submodule 622 is configured to determine a property of each molecule in the second molecule set; and
the molecule scoring submodule 623 is configured to score each molecule in the second molecule set based on the first constraint condition, to obtain a scoring result of each molecule in the second molecule set, where the scoring result of each molecule in the second molecule set is used to adjust a generation parameter in a next iteration.

The first molecule set includes the molecules in the second molecule set generated in each iteration, or the first molecule set is a molecule in a second molecule set generated in a last iteration.

In a possible implementation, the processing module 620 includes a molecule generation submodule 621, a molecular property determining submodule 622, a molecule filtering submodule 624, and a molecule scoring submodule 623. The constraint condition includes a second constraint condition and a third constraint condition, and the generating a first molecule set based on the constraint condition includes a plurality of iterations, where in each iteration,
the molecule generation submodule 621 is configured to generate a third molecule set based on a current generation parameter, where the third molecule set includes one or more molecules;
the molecular property determining submodule 622 is configured to determine a property of each molecule in the third molecule set;
the molecule filtering submodule 624 is configured to filter the molecules in the third molecule set based on the second constraint condition, and retain a molecule when the molecule meets the second constraint condition, or discard a molecule when the molecule does not meet the second constraint condition, to obtain a fourth molecule set; and
the molecule scoring submodule 623 is configured to score each molecule in the fourth molecule set based on the third constraint condition, to obtain a scoring result of each molecule in the fourth molecule set, where the scoring result of each molecule in the fourth molecule set is used to adjust a generation parameter in a next iteration.

The first molecule set includes a molecule in the fourth molecule set obtained in each iteration, or the first molecule set is a molecule in a fourth molecule set obtained in a last iteration.

In a possible implementation, the molecular property determining submodule 622 is configured to:
determine the property of each molecule in the second molecule set based on a molecular structure or a molecular formula of each molecule in the second molecule set; and/or predict the property of each molecule in the second molecule set based on a trained molecular property prediction model; or
determine the property of each molecule in the third molecule set based on a molecular structure or a molecular formula of each molecule in the third molecule set; and/or predict the property of each molecule in the third molecule set based on a trained molecular property prediction model.

Functional modules in FIG. 12 are configured to implement the steps in the method embodiments in FIG. 4 to FIG. 9. For details, refer to the descriptions of the related content in the method embodiments in FIG. 4 to FIG. 9. For brevity of the specification, details are not described herein again.

This application further provides a molecule generation device 700. The molecule generation device 700 may be configured as the server in FIG. 1 or FIG. 2. FIG. 13 is a diagram of a structure of a molecule generation device 700 according to this application. The molecule generation device 700 includes a processor 710, a communication interface 720, and a memory 730. The processor 710, the communication interface 720, and the memory 730 may be connected to each other through an internal bus 740, or may implement communication through wireless transmission and the like.

For example, the processor 710, the communication interface 720, and the memory 730 are connected to each other through the bus 740, where the bus 740 may be a peripheral component interconnect (peripheral component interconnect, PCI) bus, an extended industry standard architecture (extended industry standard architecture, EISA) bus, or the like. The bus 740 may be classified into an address bus, a data bus, a control bus, and the like. For ease of representation, only one bold line is used to represent the bus in FIG. 13, but this does not mean that there is only one bus or only one type of bus.

The processor 710 may include at least one general-purpose processor, for example, a CPU, or a combination of a CPU and a hardware chip. The hardware chip may be an application-specific integrated circuit (application-specific integrated circuit, ASIC), a programmable logic device (programmable logic device, PLD), or a combination thereof. The PLD may be a complex programmable logic device (complex programmable logic device, CPLD), a field-programmable gate array (field-programmable gate array, FPGA), generic array logic (generic array logic, GAL), or any combination thereof. The processor 710 executes various types of digital storage instructions, for example, software or firmware programs stored in the memory 730, so that the molecule generation device 700 is enabled to provide various services of a relatively wide range.

The memory 730 is configured to store program code, and the processor 710 controls execution of the program code, to perform the steps described in the embodiments in FIG. 4 to FIG. 9. For details, refer to the related descriptions in the foregoing embodiments. Details are not described herein again.

The memory 730 may include a volatile memory, for example, a RAM. Alternatively, the memory 730 may include a nonvolatile memory, for example, a ROM or a flash memory (flash memory). Alternatively, the memory 730 may include a combination of the foregoing types.

The communication interface 720 may be a wired interface (for example, an Ethernet interface), or may be an internal interface (for example, a high-speed serial computer extended bus standard (peripheral component interconnect express, PCIE) bus interface), a wired interface (for example, an Ethernet interface), or a wireless interface (for example, a cellular network interface or a wireless local area network interface), and configured to communicate with another device or module.

The processor 710, the communication interface 720, and the like in the molecule generation device 700 may implement functions and/or steps and methods implemented by the devices in the foregoing method embodiments. For brevity, details are not described herein again. The communication module 610 in the molecule generation apparatus 600 may be located in the communication interface 720 in the molecule generation device 700. The processing module 620 may be located in the processor 710 in the molecule generation device 700. Specifically, the molecule generation submodule 621, the molecular property determining submodule 622, the molecule scoring submodule 623, and the molecule filtering submodule 624 may be located in the processor 710 in the molecule generation device 700.

It should be noted that FIG. 13 is merely a possible implementation of this embodiment of this application. During actual application, molecule generation device may further include more or fewer components. This is not limited herein. For content that is not shown or described in this embodiment of this application, refer to the related descriptions in the foregoing method embodiments. Details are not described herein again.

This application further provides a computer-readable storage medium, including computer program instructions. When the computer program instructions are executed by a molecule generation device, the molecule generation device performs some or all of the steps described in the foregoing molecule generation method embodiments.

This application further provides a computer program product, including program instructions. When the program instructions are run by a molecule generation device, the molecule generation device is enabled to perform some or all of the steps described in the foregoing molecule generation method embodiments.

In the foregoing embodiments, the description of each embodiment has respective focuses. For a part that is not described in detail in an embodiment, refer to the related descriptions in other embodiments.

All or some of the foregoing embodiments may be implemented by using software, hardware, or any combination thereof. When the software is used to implement the embodiments, all or a part of the embodiments may be implemented in a form of the computer program product. The computer program product may include code. When the computer program product is read and executed by a computer, some or all steps of the method recorded in the foregoing method embodiments may be implemented. The computer may be a general-purpose computer, a dedicated computer, a computer network, or another programmable apparatus. The computer instructions may be stored in a computer-readable storage medium, or may be transmitted from a computer-readable storage medium to another computer-readable storage medium. For example, the computer instructions may be transmitted from a website, computer, server, or data center to another website, computer, server, or data center in a wired (for example, a coaxial cable, an optical fiber, or a digital subscriber line) or wireless (for example, infrared, radio, or microwave) manner. The computer-readable storage medium may be any usable medium accessible by the computer, or a data storage device, such as a server or a data center, integrating one or more usable media. The usable medium may be a magnetic medium (for example, a floppy disk, a hard disk, or a magnetic tape), an optical medium, a semiconductor medium, or the like.

Sequence adjustment, combination, or deletion may be performed on steps in the method in embodiments of this application based on an actual requirement. The units in the apparatus in embodiments of this application may be divided, combined, or deleted based on the actual requirement.

Embodiments of this application are described in detail above. The principle and implementation of this application are described herein through specific examples. The description about embodiments of this application is merely provided to help understand the method and core ideas of this application. In addition, a person of ordinary skill in the art can make variations and modifications to this application in terms of the specific implementations and application scopes according to the ideas of this application. Therefore, the content of specification shall not be construed as a limit to this application.

## Claims

1. A molecule generation method, comprising:
receiving a constraint condition entered by a user on a terminal, wherein the constraint condition indicates a condition that a property of a molecule needs to meet, and the property of the molecule comprises any one or more of a molecular weight, water solubility, lipid solubility, bioactivity, synthesizability, a docking binding affinity for a specific molecule, a similarity to a source molecule, a comprised specific substructure, pharmacokinetics, and toxicity of the molecule;
generating a first molecule set based on the constraint condition, wherein the first molecule set comprises one or more molecules; and
returning information about the one or more molecules in the first molecule set to the terminal.

2. The method according to claim 1, wherein the constraint condition comprises a first constraint condition, and the generating a first molecule set based on the constraint condition comprises a plurality of iterations, wherein
in each iteration, the method comprises:
generating a second molecule set based on a current generation parameter, wherein the second molecule set comprises one or more molecules;
determining a property of each molecule in the second molecule set; and
scoring each molecule in the second molecule set based on the first constraint condition, to obtain a scoring result of each molecule in the second molecule set, wherein the scoring result of each molecule in the second molecule set is used to adjust a generation parameter in a next iteration, wherein
the first molecule set comprises the molecules in the second molecule set generated in each iteration, or the first molecule set is a molecule in a second molecule set generated in a last iteration.

3. The method according to claim 1, wherein the constraint condition comprises a second constraint condition and a third constraint condition, and the generating a first molecule set based on the constraint condition comprises a plurality of iterations, wherein
in each iteration, the method comprises:
generating a third molecule set based on a current generation parameter, wherein the third molecule set comprises one or more molecules;
determining a property of each molecule in the third molecule set;
filtering the molecules in the third molecule set based on the second constraint condition, and retaining a molecule when the molecule meets the second constraint condition, or discarding a molecule when the molecule does not meet the second constraint condition, to obtain a fourth molecule set; and
scoring each molecule in the fourth molecule set based on the third constraint condition, to obtain a scoring result of each molecule in the fourth molecule set, wherein the scoring result of each molecule in the fourth molecule set is used to adjust a generation parameter in a next iteration, wherein
the first molecule set comprises a molecule in the fourth molecule set obtained in each iteration, or the first molecule set is a molecule in a fourth molecule set obtained in a last iteration.

4. The method according to claim 2 or 3, wherein
the determining a property of each molecule in the second molecule set comprises:
determining the property of each molecule in the second molecule set based on a molecular structure or a molecular formula of each molecule in the second molecule set; and/or predicting the property of each molecule in the second molecule set based on a trained molecular property prediction model; or
the determining a property of each molecule in the third molecule set comprises:
determining the property of each molecule in the third molecule set based on a molecular structure or a molecular formula of each molecule in the third molecule set; and/or predicting the property of each molecule in the third molecule set based on a trained molecular property prediction model.

5. A molecule generation apparatus, comprising:
a communication module, configured to receive a constraint condition entered by a user on a terminal, wherein the constraint condition indicates a condition that a property of a molecule needs to meet, and the property of the molecule comprises any one or more of a molecular weight, water solubility, lipid solubility, bioactivity, synthesizability, a docking binding affinity for a specific molecule, a similarity to a source molecule, a comprised specific substructure, pharmacokinetics, and toxicity of the molecule; and
a processing module, configured to generate a first molecule set based on the constraint condition, wherein the first molecule set comprises one or more molecules, wherein
the communication module is further configured to return information about the one or more molecules in the first molecule set to the terminal.

6. The apparatus according to claim 5, wherein the processing module comprises a molecule generation submodule, a molecular property determining submodule, and a molecule scoring submodule; and
the constraint condition comprises a first constraint condition, and the generating a first molecule set based on the constraint condition comprises a plurality of iterations, wherein in each iteration,
the molecule generation submodule is configured to generate a second molecule set based on a current generation parameter, wherein the second molecule set comprises one or more molecules;
the molecular property determining submodule is configured to determine a property of each molecule in the second molecule set; and
the molecule scoring submodule is configured to score each molecule in the second molecule set based on the first constraint condition, to obtain a scoring result of each molecule in the second molecule set, wherein the scoring result of each molecule in the second molecule set is used to adjust a generation parameter in a next iteration, wherein
the first molecule set comprises the molecules in the second molecule set generated in each iteration, or the first molecule set is a molecule in a second molecule set generated in a last iteration.

7. The apparatus according to claim 5, wherein the processing module comprises a molecule generation submodule, a molecular property determining submodule, a molecule filtering submodule, and a molecule scoring submodule; and
the constraint condition comprises a second constraint condition and a third constraint condition, and the generating a first molecule set based on the constraint condition comprises a plurality of iterations, wherein in each iteration,
the molecule generation submodule is configured to generate a third molecule set based on a current generation parameter, wherein the third molecule set comprises one or more molecules;
the molecular property determining submodule is configured to determine a property of each molecule in the third molecule set;
the molecule filtering submodule is configured to: filter the molecules in the third molecule set based on the second constraint condition, and retain a molecule when the molecule meets the second constraint condition, or discard a molecule when the molecule does not meet the second constraint condition, to obtain a fourth molecule set; and
the molecule scoring submodule is configured to score each molecule in the fourth molecule set based on the third constraint condition, to obtain a scoring result of each molecule in the fourth molecule set, wherein the scoring result of each molecule in the fourth molecule set is used to adjust a generation parameter in a next iteration, wherein
the first molecule set comprises a molecule in the fourth molecule set obtained in each iteration, or the first molecule set is a molecule in a fourth molecule set obtained in a last iteration.

8. The apparatus according to claim 6 or 7, wherein the molecular property determining submodule is configured to:
determine the property of each molecule in the second molecule set based on a molecular structure or a molecular formula of each molecule in the second molecule set; and/or predict the property of each molecule in the second molecule set based on a trained molecular property prediction model; or
determine the property of each molecule in the third molecule set based on a molecular structure or a molecular formula of each molecule in the third molecule set; and/or predict the property of each molecule in the third molecule set based on a trained molecular property prediction model.

9. A molecule generation device, comprising a memory and a processor, wherein the memory is configured to store instructions, and the processor is configured to invoke the instructions stored in the memory to perform the method according to any one of claims 1 to 4.

10. A computer storage medium, comprising program instructions, wherein when the program instructions are run on a computer, the computer is enabled to perform the method according to any one of claims 1 to 4.
